# EUROPEAN PATENT APPLICATION

(11) **EP 3 290 056 A1**
(43) Date of publication of application: **07.03.2018**
(21) Application number: 17189707.7
(22) Date of filing: 06.09.2017
(51) Int. Cl.: A61K 49/00, G01N 33/58

(54) **EXOSOME-CONJUGATED QUANTUM DOT NANOPARTICLES AND METHODS OF DETECTING EXOSOMES AND CANCER USING SAME**

(30) Priority: 06.09.2016 US 201662384027 P
(71) Applicant: Nanoco Technologies, Ltd., Manchester M13 9NT (GB)
(72) Inventor: NAASANI, Imad, Manchester M13 9NT (GB)
(74) Representative: Dauncey, Mark Peter

(57) **Abstract**

The present disclosure relates to a plurality of quantum dot nanoparticles conjugated to ligands, and in particular a plurality of quantum dot nanoparticles wherein each nanoparticle is conjugated to an exosome-specific binding ligand. The present disclosure also relates to methods of making such a plurality of conjugated quantum dot nanoparticles, methods of detecting exosomes using such a plurality of conjugated quantum dot nanoparticles and methods of detecting exosomes using such a plurality of conjugated quantum dot nanoparticles.

## Description

### FIELD OF THE INVENTION

Embodiments disclosed herein relate to a plurality of quantum dot nanoparticles conjugated to ligands, and in particular a plurality of quantum dot nanoparticles wherein each nanoparticle is conjugated to an exosome-specific binding ligand. Embodiments also include methods of making such a plurality of conjugated quantum dot nanoparticles, methods of detecting exosomes using such a plurality of conjugated quantum dot nanoparticles and methods of detecting exosomes using such a plurality of conjugated quantum dot nanoparticles.

### BACKGROUND OF THE INVENTION

It is well known that cancer is a remarkably chaotic and unpredictable disease. Once it passes the latency period of growth, it grows and invades exponentially. Therefore, early diagnosis of cancers, particularly those with poor prognoses, is a major determinant for survival rates and remission. Pancreatic ductal adenocarcinoma is a typical example of tumors with bad prognoses where mortality rates are nearly matching with incidence rates after 5 years of diagnosis. In recent years, researchers noticed that exosomes, 100-200 nm vesicles released from various cell types and tissues, are not simply "cell debris" as thought traditionally. It has been shown that exosomes have very important roles in cell communication, homeostasis, and tumorigenesis. Many studies have shown that exosomes released from inflammatory or tumor tissues have specific cargo and surface compositions. Detection of exosomes execrated from disease tissues is sought as a universal diagnostic tool. The main challenge however is the lack of an easy method for enrichment, isolation and detection.

Current isolation and detection methods are based on multiple steps of ultrafiltration and centrifugation. These steps are then followed by detection using immune assays. Additional challenges are related to the fact that most exosomes from various tissues can share overlapping markers, causing lack of specificity and high levels of signal noise. These challenges are slowing the development and realization of diagnostic kits based on exosomes.

There has been substantial interest in the preparation and characterization of particles with dimensions, for example in the range 2-50 nm, often referred to as quantum dots or nanocrystals. Quantum dots (QDs) are fluorescent nanoparticles with unique optical properties including broad-range excitation, size-tunable emission, narrow emission bandwidth, enhanced brightness (due to high extinction coefficient), photo-stability, multiplexing capabilities, and simultaneous multiple emissions using a single source of excitation. Unlike normal fluorescent dyes, the unique properties of QDs enable several potential medical applications including unmet diagnostics, clinical imaging, targeted drug delivery, and photodynamic therapy.

There is a need for new methods for detecting exosomes and for detecting cancer, *in vitro* and *in vivo* (e.g., in real time).

### SUMMARY OF THE INVENTION

Embodiments disclosed include a plurality of quantum dot nanoparticles that may be used for detecting exosomes and detecting cancer (e.g., pancreatic cancer, lung cancer, bladder cancer).

Embodiments disclosed include a plurality of quantum dot nanoparticles, wherein each nanoparticle is bonded (e.g., covalently bonded or physically bonded (by ion pairing or van der Waals interactions) to an exosome-specific binding ligand, by an amide, ester, thioester, or thiol anchoring group directly on the inorganic surface of the quantum dot nanoparticle, or on an organic corona layer that is used to render the nanoparticles water soluble and biocompatible. The water soluble quantum dot nanoparticle in certain embodiments includes a core of one semiconductor material and at least one shell of a different semiconductor material in some embodiments while in other embodiments the water soluble quantum dot nanoparticle includes an alloyed semiconductor material having a bandgap value that increases outwardly by compositionally graded alloying. The embodiments are useful for detecting exosomes and for detecting cancer, *in vitro* and *in vivo* (e.g., in real time).

In certain embodiments the light responsive quantum dot (QD) includes water soluble QD nanoparticles having a ligand interactive agent and a surface modifying ligand. The water soluble QD nanoparticle may be formed by chemical addition of the ligand interactive agent and the surface modifying ligand to the QD in a solution comprising hexamethoxymethylmelamine. In particular embodiments the ligand interactive agent is a C₈-C₂₀ fatty acid, cholesterol or esters thereof, while the surface modifying ligand is a monomethoxy polyethylene oxide.

In one embodiment, each quantum dot nanoparticle in the plurality is conjugated to a specific exosome-specific binding ligand based on the fluorescence wavelength of the quantum dot (such as, e.g., green, yellow or red). Once bound to a specific exosome, upon fluorescence, a unique emission code (a unique emission fingerprint) will be obtained that may be associated with that type of exosome and a particular type of cancer that the specific exosome is released from.

In one embodiment, each quantum dot nanoparticle in the plurality described herein is covalently linked to the exosome-specific binding ligand via an amide bond.

In one embodiment, each quantum dot nanoparticle in the plurality comprises, a core semiconductor material, and an outer layer, wherein the outer layer comprises a corona of organic coating (a functionalization organic coating) to render the particles water soluble and bio compatible, and an exosome-specific binding ligand. In one embodiment, each quantum dot nanoparticle comprises a plurality of shells of semiconductor material, the outer shell comprising an outer layer, wherein the outer layer comprises a corona of organic coating (a functionalization organic coating) to render the particles water soluble and bio compatible, and an exosome-specific binding ligand.

In one embodiment, each quantum dot nanoparticle in the plurality comprises: an alloyed quantum dot and an exosome-specific binding ligand.

In one embodiment, each quantum dot nanoparticle in the plurality comprises: a doped quantum dot and an exosome-specific binding ligand.

In one embodiment, the exosome-specific binding ligand may be any synthetic or naturally occurring exosome-specific binding agent. For example, the exosome-specific binding ligand may be selected from antibodies, fragments of antibodies, synthetic peptides, aptamers, synthetic nucleic acids, and any combination thereof. Suitable exosome-specific binding ligands include, but are not limited to, ligands against (e.g., ligands specific for) Glypican-1, CEA, CA 19-9, mesothelin, PD-L1, telomerase, and any combination thereof. In one embodiment, the exosome-specific binding ligand is a Glypican-1 antibody, a Glypican-1 tagging molecule, or a combination thereof.

In one embodiment, each quantum dot nanoparticle in the plurality is conjugated to a specific exosome-specific binding ligand based on the fluorescence wavelength of the quantum dot (such as, e.g., green, yellow or red). It should be understood that each quantum dot nanoparticle in the plurality may be individually tuned to emit at a specific wavelength across the whole visible spectrum, and beyond.

In one embodiment of any of the quantum dot nanoparticles in the pluralities described herein, the nanoparticle comprises a II-VI material, a III-V material, or I-III-IV material or any alloy or doped derivative thereof.

In one embodiment, any of the quantum dot nanoparticles in the pluralities described herein are associated with an emission spectrum ranging from about 350 nm to about 1000 nm.

In one embodiment, any of the quantum dot nanoparticles in the pluralities described herein are associated with an emission spectrum ranging from about 450 nm to about 800 nm.

In an additional embodiment, any of the quantum dot nanoparticles in the pluralities described herein may further comprise a cellular uptake enhancer, (cell-penetrating peptides (CPPs like TAT, RGD, or poly arginine), a tissue penetration enhancer, (e.g., saponins, cationic lipids, Streptolysin O (SLO)), or any combination thereof. Examples of cellular uptake enhancers include, for example, trans-activating transcriptional activators (TAT), Arg-Gly-Asp (RGD) tripeptides, or poly arginine peptides. The ligand-nanoparticle conjugates can further comprise other known agents such as, for example, saponins, cationic liposomes or Streptolysin O, that can enhance cellular uptake.

In another embodiment, a method of detecting exosomes, *in vitro* or *in vivo* (e.g., in real-time), is provided. In one embodiment, the method comprises i) contacting a plurality of ligand nanoparticle conjugates (e.g., a panel of ligand nanoparticle conjugates) according to any of the embodiments described herein with an exosome, and ii) detecting light emission or light absorbance by the plurality of nanoparticle conjugates. In an additional aspect of the embodiment, the plurality of quantum dot nanoparticles is excited with a light source (e.g., fluorescence). In one embodiment, a unique emission code (a fingerprint) is obtained that may be associated with that type of exosome. In one embodiment, the emission code (signal) is read by the naked eye, e.g., using any hand-held light source (such as, e.g., a hand-held ultra-violet (UV) source). In another embodiment, the emission code (signal) is read by a digital imaging system.

In another embodiment, a method of detecting cancer (such as, e.g., pancreatic cancer, bladder, lung cancer), *in vitro* or *in vivo* (e.g., in real-time), is provided. In one embodiment, the method comprises i) contacting a plurality of ligand nanoparticle conjugates (e.g., a panel of ligand nanoparticle conjugates) according to any of the embodiments described herein with an exosome, and ii) detecting light emission or light absorbance by the plurality of nanoparticle conjugates. In an additional aspect of the embodiment, the plurality of quantum dot nanoparticles is excited with a light source (e.g., to induce fluorescence). In one embodiment, a unique emission code (a fingerprint) is obtained that may be associated with that type of cancer that the specific exosome is released from. In one embodiment, the emission code (signal) is read by the naked eye, e.g., using any hand held light source (such as, e.g., a hand-held ultra-violet (UV) source). In another embodiment, the emission code (signal) is read by a digital imaging system. In one embodiment, the method is used for the early detection of cancer, such as early detection/staging of pancreatic cancer.

In one embodiment of any of the methods described herein, the plurality of quantum dots is excited using a multi-photon (e.g., a two-photon excitation). In such an embodiment, the combined energy of two or more light beams is used to excite a particular quantum dot nanoparticle.

In one embodiment of any of the methods described herein, the exosome bound to the quantum dot nanoparticle is disrupted and the exosome cargo protein is detected.

In one embodiment of any of the methods described herein, the plurality of conjugates is used *in vitro* in a diagnostic system such as a lateral flow test device (a dip-stick type) that may be used, e.g., for simple point-of-care detection. The lateral flow test may operate as either competitive or sandwich assays. In one embodiment, the diagnostic system estimates the titer of each fluorescent marker, thereby enabling proper staging of cancers, such as pancreatic cancer.

In one embodiment, any of the methods described herein are performed in bodily fluids (e.g., blood, pancreatic juice, plasma, fine needle aspirate) and/or tissues samples *in vivo* (e.g., in real time). In one embodiment, any of the methods described herein are performed in bodily fluids and/or tissues samples taken and examined *in vitro.*

In one embodiment, the sample is enriched prior to analysis. For example, the sample may be enriched using gel filtration spin columns, or may be embedded in a lateral flow chromatography device.

In one embodiment, the sample is a fixed tissue sample. In another embodiment, the sample is in a live cell cultivated in a cell culture. In another embodiment, the plurality of ligand-nanoparticle conjugates are introduced to living tissue. In another embodiment, the plurality of ligand-nanoparticle conjugates are introduced to a mammal for real-time detection of cancer.

In another aspect, the present invention provides the use of a plurality of ligand-nanoparticle conjugates according to any of the embodiments described herein for the detection of exosomes, e.g., *in vivo* and/or *in vitro.*

In another aspect, the present invention provides the use of a plurality of ligand-nanoparticle conjugates according to any of the embodiments described herein for the detection of cancer, e.g., *in vivo* and/or *in vitro.*

In another aspect, the present invention provides a kit (e.g., a diagnostic kit) for detecting exosomes, *in vivo* and/or *in vitro,* the kit comprising a plurality of ligand-nanoparticle conjugates according to any of the embodiments described herein.

In another aspect, the present invention provides a kit (e.g., a diagnostic kit) for detecting cancer (such as, e.g., pancreatic cancer, bladder, lung cancer), *in vivo* and/or *in vitro,* the kit comprising a plurality of ligand-nanoparticle conjugates according to any of the embodiments described herein.

### BRIEF DESCRIPTION OF THE DRAWINGS

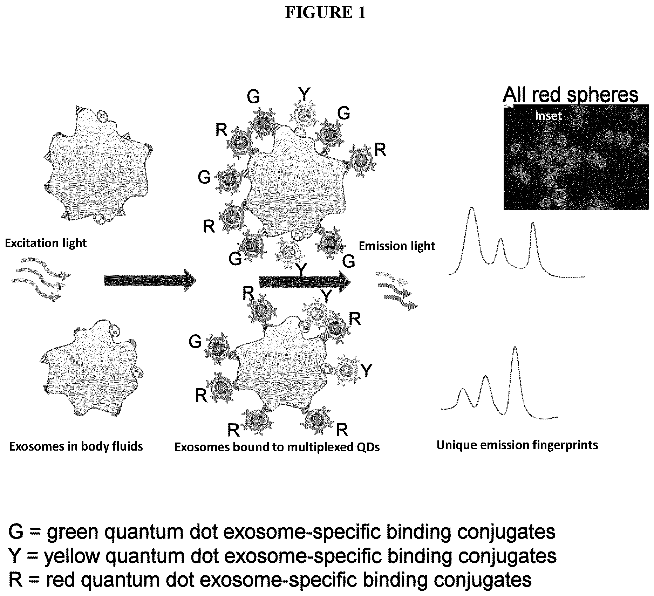

Figure 1 depicts an exemplary detection schematic wherein a red quantum dot-streptavidin conjugate was prepared and used to show specific labeling of a biotinylated polymer sphere target.

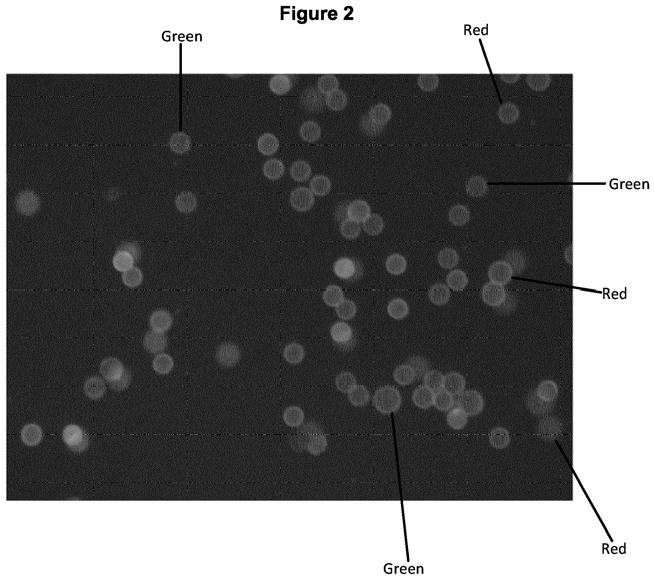

Figure 2 depicts the staining of biotinylated model polymer spheres with red and green QD-streptavidin conjugates.

### DETAILED DESCRIPTION OF THE INVENTION

Disclosed herein are Quantum Dots (QDs) conjugated with exosome specific binding ligands that have the ability to be detected upon stimulation of the QD under conditions resulting in photon emission by the QD. Also disclosed herein are certain embodiments that provide quantum dot nanoparticles (QDs) that feature high safety and biocompatibility profiles and are conjugated with exosome specific ligands. In certain embodiments, the QD is engineered as a conjugate of biocompatible, non-toxic, fluorescent quantum dot nanoparticles (QDs).

ABBREVIATIONS: To facilitate the understanding of this invention, and for the avoidance of doubt in construing the claims herein, a number of terms are defined below. Terms defined herein have meanings as commonly understood by a person of ordinary skill in the areas relevant to the present invention. The terminology used to describe specific embodiments of the invention does not delimit the invention, except as outlined in the claims.
- DCC: dicyclohexylcarbodiimide
- DCM: dichloromethane
- DIC: diisopropylcarbodiimide
- EDC: 1-ethyl-3-(-3-dimethylaminopropyl) carbodiimide hydrochloride
- HMMM: hexamethoxymethylmelamine
- In(MA)₃: indium myristate
- QD: Quantum Dots
- sulfo-NHS: sulfo derivative of N-hydroxysuccinimide
- SMCC: succinimidyl 4-(N-maleimidomethyl)cyclohexane-1-carboxylate
- (TMS)₃P: *tris*(trimethylsilyl) phosphine

The terms such as "a," "an," and "the" are not intended to refer to a singular entity unless explicitly so defined, but include the general class of which a specific example may be used for illustration. The use of the terms "a" or "an" when used in conjunction with "comprising" in the claims and/or the specification may mean "one" but may also be consistent with "one or more," "at least one," and/or "one or more than one."

The use of the term "or" in the claims is used to mean "and/or" unless explicitly indicated to refer to alternatives as mutually exclusive. Thus, unless otherwise stated, the term "or" in a group of alternatives means "any one or combination of" the members of the group. Further, unless explicitly indicated to refer to alternatives as mutually exclusive, the phrase "A, B, and/or C" means embodiments having element A alone, element B alone, element C alone, or any combination of A, B, and C taken together.

Similarly, for the avoidance of doubt and unless otherwise explicitly indicated to refer to alternatives as mutually exclusive, the phrase "at least one of" when combined with a list of items, means a single item from the list or any combination of items in the list. For example, and unless otherwise defined, the phrase "at least one of A, B and C," means "at least one from the group A, B, C, or any combination of A, B and C." Thus, unless otherwise defined, the phrase requires one or more, and not necessarily not all, of the listed items.

The terms "comprising" (and any form thereof such as "comprise" and "comprises"), "having" (and any form thereof such as "have" and "has"), "including" (and any form thereof such as "includes" and "include") or "containing" (and any form thereof such as "contains" and "contain") are inclusive or open-ended and do not exclude additional, unrecited elements or method steps.

The term "effective" as used in the specification and claims, means adequate to provide or accomplish a desired, expected, or intended result.

The terms "about" or "approximately" are defined as being close to as understood by one of ordinary skill in the art, and in one non-limiting embodiment the terms are defined to be within 10%, within 5%, within 1%, and in certain aspects within 0.5%.

In certain embodiments, the exosome specific binding ligand is an antibody that recognizes exosome epitopes. As used herein the term "antibody" includes both intact immunoglobulin molecules as well as portions, fragments, and derivatives thereof, such as, for example, Fab, Fab', F(ab')₂, Fv, Fsc, CDR regions, or any portion of an antibody that is capable of binding an antigen or epitope including chimeric antibodies that are bi-specific or that combine an antigen binding domain originating with an antibody with another type of polypeptide. The term antibody includes monoclonal antibodies (mAb), chimeric antibodies, humanized antibodies, as well as fragments, portions, regions, or derivatives thereof, provided by any known technique including but not limited to, enzymatic cleavage and recombinant techniques. The term "antibody" as used herein also includes single-domain antibodies (sdAb) and fragments thereof that have a single monomeric variable antibody domain (V_{H}) of a heavy-chain antibody. sdAb, which lack variable light (V_{L}) and constant light (C_{L}) chain domains are natively found in camelids (V_{H}H) and cartilaginous fish (V_{NAR}) and are sometimes referred to as "Nanobodies" by the pharmaceutical company Ablynx who originally developed specific antigen binding sdAb in llamas. The modifier "monoclonal" indicates the character of the antibody as being obtained from a substantially homogeneous population of antibodies, and is not to be construed as requiring production of the antibody by any particular method.

QDs are fluorescent semiconductor nanoparticles with unique optical properties. QD represent a particular very small size form of semiconductor material in which the size and shape of the particle results in quantum mechanical effects upon light excitation. Generally, larger QDs such as having a radius of 5-6nm will emit longer wavelengths in orange or red emission colors and smaller QDs such as having a radius of 2-3nm emit shorter wavelengths in blue and green colors, although the specific colors and sizes depend on the composition of the QD. Quantum Dots shine around 20 times brighter and are many times more photo-stable than any of the conventional fluorescent dyes (like indocyanine green (ICG)). Importantly, QD residence times are longer due to their chemical nature and nano-size. QDs can absorb and emit much stronger light intensities. In certain embodiments, the QD can be equipped with more than one binding tag, forming bi- or tri- specific nano-devices. The unique properties of QDs enable several medical applications that serve unmet needs.

In embodiments presented herein, the QDs are functionalized to present a hydrophilic outer layer or corona that permits use of the QDs in the aqueous environment, such as, for example, *in vivo* and *in vitro* applications in living cells. Such QDs are termed water soluble QDs.

In one embodiment the QDs may be surface equipped with a conjugation capable function (COOH, OH, NH₂, SH, azide, alkyne). In one exemplified embodiment, the water soluble non-toxic QD is or becomes carboxyl functionalized. For example, the COOH-QD may be linked to the amine terminus of a targeting antibody using a carbodiimide linking technology employing water-soluble 1-ethyl-3-(-3-dimethylaminopropyl) carbodiimide hydrochloride (EDC). The carboxyl functionalized QD is mixed with EDC to form an active O-acylisourea intermediate that is then displaced by nucleophilic attack from primary amino groups on the monoclonal antibody in the reaction mixture. If desired, a sulfo derivative of N-hydroxysuccinimide (sulfo-NHS) is added during the reaction with the primary amine bearing antibody. With the sulfo-NHS addition, the EDC couples NHS to carboxyls, forming an NHS ester that is more stable than the O-acylisourea intermediate while allowing for efficient conjugation to primary amines at physiologic pH. In either event, the result is a covalent bond between the QD and the antibody. Other chemistries like Suzuki-Miyaura cross-coupling, (succinimidyl 4-(N-maleimidomethyl)cyclohexane-1-carboxylate) (SMCC), or aldehyde based reactions may alternatively be used.

Methods of synthesizing core and core-shell nanoparticles are disclosed, for example, in co-owned United States Patent Nos. 7,867,556, 7,867,557, 7,803,423, 7,588,828, and 6,379,635. The contents of each of the forgoing patents are hereby incorporated by reference, in their entirety. U.S. Patent Nos. 9,115,097, 8,062,703, 7,985,446, 7,803,423, and 7,588,828, and U.S. Publication Nos. 2010/0283005, 2014/0264196, 2014/0277297 and 2014/0370690, the entire contents of each of which are hereby incorporated by reference, describe methods of producing large volumes of high quality monodisperse quantum dots.

In one embodiment, a core/shell particle is utilized having a central region or "core" of at least one semiconductor composition buried in or coated by one or more outer layers or "shell" of distinctly different semiconductor compositions. As an example, the core may be comprised of an alloy of In, P, Zn and S such as is formed by the description of Example 1 involving molecular seeding of indium-based QDs over a ZnS molecular cluster followed by formation of a shell of ZnS.

In still other embodiments, the water soluble QD nanoparticle employed comprises an alloyed semiconductor material having a bandgap value or energy (Eg) that increases outwardly by graded alloying in lieu of production of a core/shell QD. The band gap energy (Eg), is the minimum energy required to excite an electron from the ground state valence energy band into the vacant conduction energy band.

The graded alloy QD composition is considered "graded" in elemental composition from at or near the center of the particle to the outermost surface of the QD rather than formed as a discrete core overlaid by a discrete shell layer. An example would be an In₁-ₓP₁-_{y}ZnₓS_{y}, graded alloy QD wherein the x and y increase gradually from 0 to 1 from the center of the QD to the surface. In such example, the band gap of the QD would gradually change from that of pure InP towards the center to that of a larger band gap value of pure ZnS at the surface. Although the band gap is dependent on particle size, the band gap of ZnS is wider than that of InP such that the band gap of the graded alloy would gradually increase from an inner aspect of the QD to the surface.

A one-pot synthesis process may be employed as a modification of the molecular seeding process described in Example 1 herein. This may be achieved by gradually decreasing the amounts of indium myristate and (TMS)₃P added to the reaction solution to maintain particle growth, while adding increasing amounts of zinc and sulfur precursors during a process such as is described for generation of the "core" particle of Example 1. Thus, in one example a dibutyl ester and a saturated fatty acid are placed into a reaction flask and degassed with heating. Nitrogen is introduced and the temperature is increased. A molecular cluster, such as for example a ZnS molecular cluster [Et₃NH]₄ [Zn₁₀S₄(SPh)₁₆], is added with stirring. The temperature is increased as graded alloy precursor solutions are added according to a ramping protocol that involves addition of gradually decreasing concentrations of a first semiconductor material and gradually increasing concentrations of a second semiconductor material. For example, the ramping protocol may begin with additions of indium myristate (In(MA)₃) and tris(trimethylsilyl) phosphine (TMS)₃P dissolved in a dicarboxylic acid ester (such as for example di-n-butylsebacate ester) wherein the amounts of added In(MA)₃ and (TMS)₃P gradually decrease over time to be replaced with gradually increasing concentration of sulfur and zinc compounds such as (TMS)₂S and zinc acetate. As the added amounts of In(MA)₃ and (TMS)₃P decrease, gradually increasing amounts of (TMS)₂S dissolved in a saturated fatty acid (such as for example myristic or oleic acid) and a dicarboxylic acid ester (such as di-n-butyl sebacate ester) are added together with the zinc acetate. The following reactions will result in the increasing generation of ZnS compounds. As the additions continue, QD particles of a desired size with an emission maximum gradually increasing in wavelength are formed wherein the concentrations of InP and ZnS are graded with the highest concentrations of InP towards a center of the QD particle and the highest concentrations of ZnS on an outer layer of the QD particle. Further additions to the reaction are stopped when the desired emission maximum is obtained and the resultant graded alloy particles are left to anneal followed by isolation of the particles by precipitation and washing.

A nanoparticle's compatibility with a medium as well as the nanoparticle's susceptibility to agglomeration, photo-oxidation and/or quenching, is mediated largely by the surface composition of the nanoparticle. The coordination about the final inorganic surface atoms in any core, core-shell or core-multi shell nanoparticle may be incomplete, with highly reactive "dangling bonds" on the surface, which can lead to particle agglomeration. This problem is overcome by passivating (capping) the "bare" surface atoms with protecting organic groups, referred to herein as capping ligands or a capping agent. The capping or passivating of particles prevents particle agglomeration from occurring but also protects the particle from its surrounding chemical environment and provides electronic stabilization (passivation) to the particles, in the case of core material. Capping ligands may be but are not limited to a Lewis base bound to surface metal atoms of the outermost inorganic layer of the particle. The nature of the capping ligand largely determines the compatibility of the nanoparticle with a particular medium. The capping ligand may be selected depending on desired characteristics. Types of capping ligands that may be employed include thiol groups, carboxyl, amine, phosphine, phosphine oxide, phosphonic acid, phosphinic acid, imidazole, OH, thio ether, and calixarene groups. With the exception of calixarenes, all of these capping ligands have head groups that can form anchoring centers for the capping ligands on the surface of the particle. The body of the capping ligand can be a linear chain, cyclic, or aromatic. The capping ligand itself can be large, small, oligomeric or polydentate. The nature of the body of the ligand and the protruding side that is not bound onto the particle, together determine if the ligand is hydrophilic, hydrophobic, amphiphilic, negative, positive or zwitterionic.

In many quantum dot materials, the capping ligands are hydrophobic (for example, alkyl thiols, fatty acids, alkyl phosphines, alkyl phosphine oxides, and the like). Thus, the nanoparticles are typically dispersed in hydrophobic solvents, such as toluene, following synthesis and isolation of the nanoparticles. Such capped nanoparticles are typically not dispersible in more polar media. If surface modification of the QD is desired, the most widely used procedure is known as ligand exchange. Lipophilic ligand molecules that coordinate to the surface of the nanoparticle during core synthesis and/or shelling procedures may subsequently be exchanged with a polar/charged ligand compound. An alternative surface modification strategy intercalates polar/charged molecules or polymer molecules with the ligand molecules that are already coordinated to the surface of the nanoparticle. However, while certain ligand exchange and intercalation procedures render the nanoparticle more compatible with aqueous media, they may result in materials of lower quantum yield (QY) and/or substantially larger size than the corresponding unmodified nanoparticle.

For *in vivo* and *in vitro* purposes, QDs with low toxicity profiles are desirable if not required. Thus, for some purposes, the quantum dot nanoparticle is preferably substantially free of toxic heavy metals such as cadmium, lead and arsenic (e.g., contains less than 5 wt. %, such as less than 4 wt. %, less than 3 wt. %, less than 2 wt. %, less than 1 wt. %, less than 0.5 wt. %, less than 0.1 wt. %, less than 0.05 wt. %, or less than 0.01 wt. % of heavy metals such as cadmium, lead and arsenic) or is free of heavy metals such as cadmium, lead and arsenic. In one embodiment, reduced toxicity QD that lack heavy metals such as cadmium, lead, and arsenic are provided.

The unique properties of QDs enable several potential medical applications including unmet *in vitro* and *in vivo* diagnostics in living cells. One of the major concerns regarding the medical applications of QDs has been that the majority of research has focused on QDs containing toxic heavy metals such as cadmium, lead or arsenic. The biologically compatible and water-soluble heavy metal-free QDs described herein can safely be used in medical applications both *in vitro* and *in vivo.* In certain embodiments, *in vivo* compatible water dispersible cadmium-free QDs are provided that have a hydrodynamic size of 10-20 nm (within the range of the dimensional size of a full IgG2 antibody). In one embodiment, the *in vivo* compatible water dispersible cadmium-free QDs are produced in accordance with the procedures set out in Examples 1 and 2 herein. In certain embodiments, the *in vivo* compatible water dispersible cadmium-free QDs are carboxyl functionalized and further derivatized with a ligand binding moiety.

Examples of cadmium, lead and arsenic free nanoparticles include nanoparticles comprising semiconductor materials, e.g., ZnS, ZnSe, ZnTe, InP, InSb, AlP, AlS, AlSb, GaN, GaP, GaSb, PbS, PbSe, AgInS₂, CuInS₂, Si, Ge, and alloys and doped derivatives thereof, particularly, nanoparticles comprising cores of one of these materials and one or more shells of another of these materials.

In certain embodiments, non-toxic QD nanoparticles are surface modified to enable them to be water soluble and to have surface moieties that allow derivatization by exposing them to a ligand interactive agent to effect the association of the ligand interactive agent and the surface of the QD. The ligand interactive agent can comprise a chain portion and a functional group having a specific affinity for, or reactivity with, a linking/crosslinking agent, as described below. The chain portion may be, for example, an alkane chain. Examples of functional groups include nucleophiles such as thio groups, hydroxyl groups, carboxamide groups, ester groups, and a carboxyl groups. The ligand interactive agent may, or may not, also comprise a moiety having an affinity for the surface of a QD. Examples of such moieties include thiols, amines, carboxylic groups, and phosphines. If the ligand interactive group does not comprise such a moiety, the ligand interactive group can associate with the surface of the nanoparticle by intercalating with capping ligands. Examples of ligand interactive agents include C₈₋₂₀ fatty acids and esters thereof, such as for example isopropyl myristate.

It should be noted that the ligand interactive agent may be associated with a QD nanoparticle simply as a result of the processes used for the synthesis of the nanoparticle, obviating the need to expose nanoparticle to additional amounts of ligand interactive agents. In such case, there may be no need to associate further ligand interactive agents with the nanoparticle. Alternatively, or in addition, QD nanoparticle may be exposed to a ligand interactive agent after the nanoparticle is synthesized and isolated. For example, the nanoparticle may be incubated in a solution containing the ligand interactive agent for a period of time. Such incubation, or a portion of the incubation period, may be at an elevated temperature to facilitate association of the ligand interactive agent with the surface of the nanoparticle. Following association of the ligand interactive agent with the surface of nanoparticle, the QD nanoparticle is exposed to a linking/crosslinking agent and a surface modifying ligand. The linking/crosslinking agent includes functional groups having specific affinity for groups of the ligand interactive agent and with the surface modifying ligand. The ligand interactive agent-nanoparticle association complex can be exposed to a linking/crosslinking agent and surface modifying ligand sequentially. For example, the nanoparticle might be exposed to the linking/crosslinking agent for a period of time to effect crosslinking, and then subsequently exposed to the surface-modifying ligand to incorporate it into the ligand shell of the nanoparticle. Alternatively, the nanoparticle may be exposed to a mixture of the linking/crosslinking agent and the surface modifying ligand thus effecting crosslinking and incorporating surface-modifying ligand in a single step.

In one embodiment, quantum dot precursors are provided in the presence of a molecular cluster compound under conditions whereby the integrity of the molecular cluster is maintained and acts as a well-defined prefabricated seed or template to provide nucleation centers that react with the chemical precursors to produce high quality nanoparticles on a sufficiently large scale for industrial application.

Suitable types of quantum dot nanoparticles useful in the present invention include, but are not limited to, core materials comprising the following types (including any combination or alloys or doped derivatives thereof):
IIA-VIB (2-16) material, incorporating a first element from group 2 of the periodic table and a second element from group 16 of the periodic table and also including ternary and quaternary materials and doped materials. Suitable nanoparticle materials include, but are not limited to: MgS, MgSe, MgTe, CaS, CaSe, CaTe, SrS, SrSe, SrTe, BaS, BaSe, BaTe.
II-V material incorporating a first element from group 12 of the periodic table and a second element from group 15 of the periodic table and also including ternary and quaternary materials and doped materials. Suitable nanoparticle materials include, but are not limited to: Zn₃P₂, Zn₃As₂, Cd₃P₂, Cd₃As₂, Cd₃N₂, Zn₃N₂.
II-VI material incorporating a first element from group 12 of the periodic table and a second element from group 16 of the periodic table and also including ternary and quaternary materials and doped materials. Suitable nanoparticle materials include, but are not limited to: CdSe, CdTe, ZnS, ZnSe, ZnTe, ZnO, HgS, HgSe, HgTe, CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, CdZnSeS, CdZnSeTe, CdHgSeS, CdHgSeTe, CdHgSTe, HgZnSeS, and HgZnSeTe.
III-V material incorporating a first element from group 13 of the periodic table and a second element from group 15 of the periodic table and also including ternary and quaternary materials and doped materials. Suitable nanoparticle materials include, but are not limited to: BP, AlP, AlSb; GaN, GaP, GaSb; InN, InP, InSb, AIN, BN.
III-IV material incorporating a first element from group 13 of the periodic table and a second element from group 14 of the periodic table and also including ternary and quaternary materials and doped materials. Suitable nanoparticle materials include, but are not limited to: B₄C, Al₄C₃, Ga₄C, Si, SiC.
III-VI material incorporating a first element from group 13 of the periodic table and a second element from group 16 of the periodic table and also including ternary and quaternary materials. Suitable nanoparticle materials include, but are not limited to: Al₂S₃, Al₂Se₃, Al₂Te₃, Ga₂S₃, Ga₂Se₃, GeTe; In₂S₃, In₂Se₃, Ga₂Te₃, In₂Te₃, InTe.
IV-VI material incorporating a first element from group 14 of the periodic table and a second element from group 16 of the periodic table, and also including ternary and quaternary materials and doped materials. Suitable nanoparticle materials include, but are not limited to: PbS, PbSe, PbTe, Sb₂Te₃, SnS, SnSe, SnTe.

Nanoparticle material incorporating a first element from any group in the transition metal of the periodic table, and a second element from group 16 of the periodic table and also including ternary and quaternary materials and doped materials. Suitable nanoparticle materials include, but are not limited to: NiS, CrS, AgS, or I-III-VI material, for example, CuInS₂, CuInSe₂, CuGaS₂, AgInS₂.

The term doped nanoparticle for the purposes of specifications and claims refers to nanoparticles of the above and a dopant comprising one or more main group or rare earth elements, this most often is a transition metal or rare earth element, such as but not limited to zinc sulfide with manganese, such as ZnS nanoparticles doped with Mn⁺.

In one embodiment, the quantum dot nanoparticle is substantially free of heavy metals such as cadmium (e.g., contains less than 5 wt. %, such as less than 4 wt. %, less than 3 wt. %, less than 2 wt. %, less than 1 wt. %, less than 0.5 wt. %, less than 0.1 wt. %, less than 0.05 wt. %, or less than 0.01 wt. % of heavy metals such as cadmium) or is free of heavy metals such as cadmium.

For *in vivo* applications, heavy metal-free semi-conductor indium-based quantum dots, for example, InP quantum dots and their alloys and doped derivatives are preferred.

In an embodiment, any of the quantum dot nanoparticles described herein include a first layer including a first semiconductor material provided on the nanoparticle core. A second layer including a second semiconductor material may be provided on the first layer.

### Synthesis

The following synthesis steps may be used for conjugation. Linkers may be used to form an amide group between the carboxyl functions on the nanoparticles and the amine end groups on the exosome-specific binding ligand. Known linkers, such as a thiol anchoring groups directly on the inorganic surface of the quantum dot nanoparticle can be used. Standard coupling conditions can be employed and will be known to a person of ordinary skill in the art. For example, suitable coupling agents include, but are not limited to, carbodiimides, such as dicyclohexylcarbodiimide (DCC), diisopropylcarbodiimide (DIC), and 1-(3-dimethylaminopropyl)-3-ethylcarbodiimide hydrochloride (EDC). In one embodiment, the coupling agent is EDC.

In an example, the quantum dot nanoparticles bearing a carboxyl end group and a exosome-specific ligand may be mixed in a solvent. A coupling agent, such as EDC, may be added to the mixture. The reaction mixture may be incubated. The crude exosome-specific ligand nanoparticle conjugate may be subject to purification and/or isolated.

Standard solid state purification methods may be used. Several cycles of filtering and washing with a suitable solvent may be necessary to remove excess unreacted functionalized ligand and/or EDC.

In another aspect, one embodiment provides a process for preparing a ligand nanoparticle conjugate for according to any of the embodiments described herein. In one embodiment, the process comprises: i) coupling a quantum dot nanoparticle with an exosome-specific binding ligand to give a ligand-nanoparticle conjugate, wherein the nanoparticle comprises a core semiconductor material, and an outer layer, wherein the outer layer comprises a carboxyl group. In one embodiment, coupling step i) comprises (a) reacting a carboxyl group in the outer layer with a carbodiimide linker to activate the carboxyl group, and b) reacting the activated carboxyl group with a methylation specific binding ligand (e.g., with an amine terminus on the ligand).

In an additional embodiment, the process further comprises: ii) purifying the ligand nanoparticle conjugate. In an additional embodiment, the process further comprises: iii) isolating the ligand nanoparticle conjugate. In one embodiment, the process comprises steps i), ii) and iii).

In an additional embodiment, the process further comprises: ii) purifying the quantum dot nanoparticle. In an additional embodiment, the process further comprises: iii) isolating the specific binding nanoparticle conjugate. In one embodiment, the process comprises steps i), ii) and iii).

### EXAMPLES

The following examples are included for the sake of completeness of disclosure and to illustrate the methods of making the compositions and composites of the present invention as well as to present certain characteristics of the compositions. In no way are these examples intended to limit the scope or teaching of this disclosure.

### EXAMPLE 1

### Synthesis of Non-Toxic Quantum Dots

A molecular seeding process was used to generate non-toxic Quantum Dots (QD). Briefly, the preparation of non-functionalized indium-based quantum dots with emission in the range of 500 - 700 nm was carried out as follows: Dibutyl ester (approximately 100 ml) and myristic acid (MA) (10.06 g) were placed in a three-neck flask and degassed at ∼70°C under vacuum for 1 h. After this period, nitrogen was introduced and the temperature was increased to ∼90°C. Approximately 4.7 g of a ZnS molecular cluster [Et₃NH]₄ [Zn₁₀S₄(SPh)₁₆] was added, and the mixture was stirred for approximately 45 min. The temperature was then increased to ∼100°C, followed by the drop-wise additions of In(MA)₃ (1M, 15 ml) followed by tris(trimethylsilyl )phosphine (TMS)₃P (1M, 15 ml). The reaction mixture was stirred while the temperature was increased to ∼140°C. At 140°C, further drop-wise additions of indium myristate (In(MA)₃) dissolved in di-n-butylsebacate ester (1M, 35 ml) (left to stir for 5 min) and (TMS)₃P dissolved in di-n-butylsebacate ester (1M, 35 ml) were made. The temperature was then slowly increased to 180°C, and further dropwise additions of In(MA)₃ (1M, 55 ml) followed by (TMS)₃P (1M, 40 ml) were made. By addition of the precursor in this manner, indium-based particles with an emission maximum gradually increasing from 500 nm to 720 nm were formed. The reaction was stopped when the desired emission maximum was obtained and left to stir at the reaction temperature for half an hour. After this period, the mixture was left to anneal for up to approximately 4 days (at a temperature -20-40 °C below that of the reaction). A UV lamp was also used at this stage to aid in annealing.

The particles were isolated by the addition of dried degassed methanol (approximately 200 ml) via cannula techniques. The precipitate was allowed to settle and then methanol was removed via cannula with the aid of a filter stick. Dried degassed chloroform (approximately 10 ml) was added to wash the solid. The solid was left to dry under vacuum for 1 day. This procedure resulted in the formation of indium-based nanoparticles on ZnS molecular clusters. In further treatments, the quantum yields of the resulting indium-based nanoparticles were further increased by washing in dilute hydrofluoric acid (HF). The quantum efficiencies of the indium-based core material ranged from approximately 25% - 50%. This composition is considered an alloy structure comprising In, P, Zn and S.

Growth of a ZnS shell: A 20 ml portion of the HF-etched indium-based core particles was dried in a three-neck flask. 1.3 g of myristic acid and 20 ml di-n-butyl sebacate ester were added and degassed for 30 min. The solution was heated to 200°C, and 2 ml of 1 M (TMS)₂S was added drop-wise (at a rate of 7.93 ml/h). After this addition was complete, the solution was left to stand for 2 min, and then 1.2 g of anhydrous zinc acetate was added. The solution was kept at 200°C for 1 hr and then cooled to room temperature. The resulting particles were isolated by adding 40 ml of anhydrous degassed methanol and centrifuging. The supernatant liquid was discarded, and 30 ml of anhydrous degassed hexane was added to the remaining solid. The solution was allowed to settle for 5 h and then centrifuged again. The supernatant liquid was collected and the remaining solid was discarded. The quantum efficiencies of the final non-functionalized indium-based nanoparticle material ranged from approximately 60%-90% in organic solvents.

### EXAMPLE 2

### Water Soluble Surface Modified QDs

Provided herein is one embodiment of a method for generating and using melamine hexamethoxymethylmelamine (HMMM) modified fluorescent nanoparticles as drug delivery vehicles. The unique melamine-based coating presents excellent biocompatibility, low toxicity and very low non-specific binding. These unique features allow a wide range of biomedical applications both *in vitro* and *in vivo.*

One example of preparation of a suitable water soluble nanoparticle is provided as follows: 200 mg of cadmium-free quantum dot nanoparticles with red emission at 608 nm having as a core material an alloy comprising indium and phosphorus with Zn-containing shells as described in Example 1 was dispersed in toluene (1 ml) with isopropyl myristate (100 microliters). The isopropyl myristate is included as the ligand interactive agent. The mixture was heated at 50°C for about 1-2 minutes then slowly shaken for 15 hours at room temperature. A toluene solution (4 ml) of hexamethoxymethylmelamine (HMMM) (CYMEL 303, available from Cytec Industries, Inc., West Paterson, NJ) (400 mg), monomethoxy polyethylene oxide (CH₃O-PEG₂₀₀₀-OH) (400 mg), and salicylic acid (50 mg) was added to the nanoparticle dispersion. The salicylic acid that is included in the functionalization reaction plays three roles, as a catalyst, a crosslinker, and a source for COOH. Due in part to the preference of HMMM for OH groups, many COOH groups provided by the salicylic acid remain available on the QD after crosslinking.

HMMM is a melamine-based linking/crosslinking agent having the following structure:

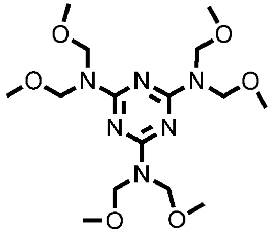

HMMM can react in an acid-catalyzed reaction to crosslink various functional groups, such as amides, carboxyl groups, hydroxyl groups, and thiols.

The mixture was degassed and refluxed at 130°C for the first hour followed by 140°C for 3 hours while stirring at 300 rpm with a magnetic stirrer. During the first hour a stream of nitrogen was passed through the flask to ensure the removal of volatile byproducts generated by the reaction of HMMM with nucleophiles. The mixture was allowed to cool to room temperature and stored under inert gas. The surface-modified nanoparticles showed little or no loss in fluorescence quantum yield and no change in the emission peak or full width at half max (FWHM) value, compared to unmodified nanoparticles. An aliquot of the surface-modified nanoparticles was dried under vacuum and deionized water was added to the residue. The surface-modified nanoparticles dispersed well in the aqueous media and remained dispersed permanently. In contrast, unmodified nanoparticles could not be suspended in the aqueous medium. The fluorescence quantum yield of the surface-modified nanoparticles according to the above procedure is 40 - 50 %. In typical batches, a quantum yield of 47% ± 5% is obtained.

In another embodiment, cadmium-free quantum dot nanoparticles (200 mg) with red emission at 608 nm were dispersed in toluene (1 ml) with cholesterol (71.5 mg). The mixture was heated at 50° C for about 1-2 minutes then slowly shaken for 15 hours at room temperature. A toluene solution (4 ml) of HMMM (Cymel 303) (400 mg), monomethoxy polyethylene oxide (CH₃O-PEG₂₀₀₀-OH) (400 mg), guaifenesin (100mg), dichloromethane (DCM) (2mL) and salicylic acid (50 mg) was added to the nanoparticle dispersion.

As used herein the compound "guaifenesin" has the following chemical structure:

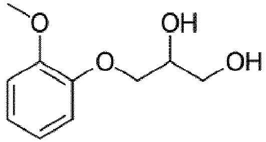

As used herein the compound "salicylic acid" has the following chemical structure:

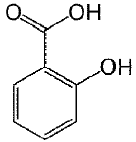

The mixture was degassed and refluxed at 140° C for 4 hours while stirring at 300 rpm with a magnetic stirrer. As with the prior procedure, during the first hour a stream of nitrogen was passed through the flask to ensure the removal of volatile byproducts generated by the reaction of HMMM with nucleophiles. The mixture was allowed to cool to room temperature and stored under inert gas. An aliquot of the surface-modified nanoparticles was dried under vacuum and deionized water was added to the residue. The pH of the solution was adjusted to 6.5 using a 100 mM KOH solution and the excess non reacted material was removed by three cycles of ultrafiltration using Amicon filters (30kD) [Merck KGAA, Frankfurter Strasse, 250 D-64293, Darmstadt, Germany]. The final aqueous solution was kept refrigerated until use.

It is noteworthy that traditional methods for modifying nanoparticles to increase their water solubility (e.g., ligand exchange with mercapto-functionalized water soluble ligands) are ineffective under mild conditions to render the nanoparticles water soluble. Under harsher conditions, such as heat and sonication, the fraction that becomes water soluble has very low quantum yield (QY <20%). The instant method, in contrast, provides water soluble nanoparticles with high quantum yield. As defined herein, a high quantum yield is equal to or greater than 40%. In certain embodiments, a high quantum yield is obtained of equal to or greater than 45%. The surface-modified nanoparticles prepared as in this example also disperse well and remain permanently dispersed in other polar solvents, including ethanol, propanol, acetone, methylethylketone, butanol, tripropylmethylmethacrylate, or methylmethacrylate.

### EXAMPLE 3

### Water soluble QD Including Targeting Ligands

In certain embodiments, the water soluble QD is modified to include targeting ligands that are added to the QD. Thus, in one embodiment quantum dot nanoparticles are synthesized that are non-toxic and water soluble (biocompatible) and are surface equipped with a conjugation capable function (COOH, OH, NH₂, SH, azide, alkyne). By virtue of the functional groups that can be added to the QD, such as for example the COOH functional group provided in Example 2 herein, the QD can be modified to include a targeting ligand that allows the QD to selectively identify exosomes in samples, cells and tissues. The targeting ligand modified QD is the irradiated and emits light for detection.

In one exemplified embodiment, the water soluble non-toxic QD is or becomes carboxyl functionalized. The COOH-QD is linked to the amine terminus of a exosome targeting moiety such as a specific antibody using a chemical method such as for example a carbodiimide linking technology employing water-soluble 1-ethyl-3-(-3-dimethylaminopropyl) carbodiimide hydrochloride (EDC). The carboxyl functionalized QD is mixed with EDC to form an active O-acylisourea intermediate that is then displaced by nucleophilic attack from primary amino groups on the monoclonal antibody in the reaction mixture. If desired, a sulfo derivative of N-hydroxysuccinimide (sulfo-NHS) is added during the reaction with the primary amine-bearing antibody. With the sulfo-NHS addition, the EDC couples NHS to carboxyls, forming an NHS ester that is more stable than the O-acylisourea intermediate while allowing for efficient conjugation to primary amines at physiologic pH. In either event, the result is a covalent bond between the QD and the antibody. Other chemistries like Suzuki-Miyaura cross-coupling, succinimidyl 4-(N-maleimidomethyl) cyclohexane-1-carboxylate (SMCC), or aldehyde based reactions may alternatively be used.

In one embodiment, non-toxic, water-soluble quantum dots are chemically attached to an antibody directed to exosome binding sites, such as, for example, an exosome-specific binding ligand specific for Glypican-1, CA19-9, mesothelin, PD-L1, telomerase, or any combination thereof.

*Covalent conjugation of in vivo compatible water dispersible cadmium-free QD with exosome specific binding ligands:* In Eppendorf tubes, 1 mg carboxyl-functionalised, water-soluble quantum dots are mixed with 100 µl MES activation buffer (i.e. 25 µl of 40mg/ml stock into 100 µl MES). The MES buffer is prepared as a 25 mM solution (2-(N-morpholino) ethanesulfonic acid hemisodium salt (MES), Sigma Aldrich) in deionized (DI) water, pH 4.5. To this, 33 µl of a fresh EDC solution (30 mg/ml stock in DI water, 1-ethyl-3-(3-dimethylaminopropyl) carbodiimide hydrochloride (EDC), Fisher Scientific) is added and the solution is mixed. 4 µl of fresh sulfo-NHS (100 mg/ml stock, ThermoFisher Scientific, in DI water) is added and mixed. NanoSep 300K filters (PALL NanoSep 300K Omega ultrafilters) are pre-wetted in 100 µl MES. The MES/EDC/Sulfo-NHS/QD solution is added to the NanoSep 300K filter and is topped off with sufficient MES. The filter is centrifuged at 5000 rpm/15 min. The dots are re-dispersed in 50 µl activation buffer and are transferred to an Eppendorf tube containing 10 µl of methylation specific ligand. The solution is mixed well and incubated at room temperature overnight (around 16 - 18 hours). The solution is quenched with 16 µl of 6-amino caproic acid (6AC) (19.7 mg/100 mM). Note that quenching could be alternatively conducted with other compounds having a primary amine, but 6AC is selected for this embodiment because it has a COOH and can maintain the colloidal stability of the product. The solution is transferred to a pre-wetted Nanosep 300K filter (100 µl 1x PBS) and is topped-up to the 500 µl line with 1x PBS. Excess SAV is removed by three cycles of ultrafiltration using Nanosep 300K filters and 1x PBS buffer. Each cycle of centrifugation is run at 5000 rpm for 20 min with re-dispersal with -400 ul of 1x PBS after each cycle. The final concentrate is re-dispersed in 100 µl PBS.

### EXAMPLE 4

Standard conjugation chemistry may be used for conjugation. For example, a method preparing a nanoparticle exosome-specific binding ligand conjugate may include the steps of providing a nanoparticle, providing a coupling agent, providing an exosome-specific binding ligand, such as, for example, an exosome-specific binding ligand specific for Glypican-1, CA19-9, mesothelin, PD-L1, telomerase, or any combination thereof, and incubating the mixture to form a nanoparticle exosome-specific binding ligand conjugate. The mixture may then be purified and isolated to obtain a nanoparticle exosome-specific binding ligand conjugate.

The incubation conditions may be chosen to allow for formation of either an amide or an ester. It should be understood that other bonds may be formed (e.g., both covalent and noncovalent). The exosome-specific binding ligand can be conjugated with the nanoparticle either covalently, physically, ion pairing, or van der Waals interactions. The bond may be formed by an amide, ester, thioester, or thiol anchoring group directly on the inorganic surface of the quantum dot nanoparticle, or on the organic corona layer that is used to render the nanoparticles water soluble and biocompatible.

Standard incubation conditions for coupling can be employed. For example, the coupling conditions may be a solution in the range of 0.5 to 4 hours. The temperature range of the coupling conditions may be in the range of 100°C to 200°C. The coupling conditions may be constant or varied during the reaction. For example, the reaction conditions may be 130°C for one hour then raised to 140°C for three hours.

Linkers may be used to form an amide or an ester group between the carboxyl functions on the nanoparticles and either the carboxyl or the amine end groups on the exosome-specific binding ligand. Linkers or coupling agents may include benzotriazolyloxytris(dimethylamino) phosphonium Hexafluorophosphate (BOP) and carbodiimides such as dicyclohexylcarbodiimide (DCC), diisopropylcarbodiimide (DIC), and 1-(3-dimethylaminopropyl)-3-ethylcarbodiimide hydrochloride (EDC). EDC is a preferred carbodiimide to use as the amide coupling agent.

In an example, the quantum dot nanoparticles bearing a carboxyl end group and exosome-specific binding ligand may be mixed in a solvent. A coupling agent, such as EDC, may be added to the mixture. The reaction mixture may be incubated. A crude exosome-specific binding ligand - QD nanoparticle conjugate may be subjected to purification to obtain the conjugated QD nanoparticle conjugate.

Standard solid state purification method may be used. Several cycles of filtering and washing with a suitable solvent may be necessary to remove excess unreacted exosome-specific binding ligand and EDC.

The nanoparticle exosome-specific binding ligand conjugate can subsequently be introduced into a mammal or tissue for real-time imaging to determine or to diagnose a particular type of cancer that the specific exosome is released from. For example, each quantum dot nanoparticle in the plurality can be conjugated to a specific exosome-specific binding ligand based on the emission color of the quantum dot (such as, e.g., green, yellow or red). Once bound to a specific exosome, upon fluorescence, a unique emission code (a unique emission fingerprint) will be obtained that may be associated with that type of exosome and a particular type of cancer that the specific exosome is released from.

The administration of the exosome-specific binding ligand conjugate can be enteral or parenteral. For example, the exosome-specific binding ligand conjugate can be administered subcutaneously, intravenously, intramuscular, topically, and orally. Examples include bolus injections or IV infusions.

### Preparation and Analysis of Clinical Samples

Positive body fluid samples withdrawn from patients with confirmed pancreatic cancer diagnosis and stage are prepared following proper patient consent and authorization. Initially, samples from pancreatic juices, needle aspirates, biliary brushings and plasma are used as crude body fluids and mixed with the QD conjugate and the sample applied on a dot blot paper that has fixed secondary antibodies to capture the QD-exosome or exosomal protein complex. Once a signal is confirmed using fluorescence detection methods (visual, microscopy or a plate reader), the type of sample can be adopted for further analysis using a lateral flow assay device. If a weak signal is observed, then a one-step exosome enrichment step using gel filtration spin columns can be used. Additional markers may also be added to the detection system.

In another embodiment, a ligand-nanoparticle conjugate is introduced to an organism *in vivo.* Such organisms may include prokaryotic or eukaryotic organisms including mammals. Suitable exosome binding ligands of the ligand-nanoparticle conjugate include, but are not limited to, such as, for example, an exosome-specific binding ligand specific for Glypican-1, CA19-9, mesothelin, PD-L1, telomerase, or any combination thereof. The ligand-nanoparticle conjugate is allowed to contact an exosome. The ligand-nanoparticle conjugate is excited by a light source in vivo or ex vivo if a tissue sample has been removed from the organism for detection. The light emission or light absorbance by the ligand-nanoparticle conjugate is measured and quantified. The detection can be performed in real-time.

These and other advantages of the present invention will be apparent to those skilled in the art from the foregoing specification. For example, those skilled in the art will appreciate that multiple exosome ligand conjugated QDs could be introduced to a mammal or portion thereof that emit different fluorescence wavelengths that are specific for a particular type of cancer; for example, QDs emitting red could be specific to pancreatic cancer while those emitting green could be specific to another type of cancer. Accordingly, it is to be recognized by those skilled in the art that changes or modifications may be made to the above-described embodiments without departing from the broad inventive concepts of the invention. It is to be understood that this invention is not limited to the particular embodiments described herein, but is intended to include all changes and modifications that are within the scope and spirit of the invention.

## Claims

1. A plurality of quantum dot nanoparticle conjugates, wherein each nanoparticle is linked to an exosome-specific binding ligand.

2. The plurality of quantum dot nanoparticle conjugates of claim 1, wherein each quantum dot nanoparticle comprises:
a core semiconductor material, and
an outer layer,
wherein the outer layer comprises a functionalization organic coating linked to an exosome-specific binding ligand.

3. The plurality of quantum dot nanoparticle conjugates of claim 1, wherein each quantum dot nanoparticle is conjugated to a specific exosome-specific binding ligand based on the fluorescence wavelength of the quantum dot.

4. The plurality of quantum dot nanoparticle conjugates of claim 1, wherein the exosome-specific binding ligand is:
i) selected from antibodies, fragments of antibodies, synthetic peptides, aptamers, synthetic nucleic acids, and any combination thereof; or
ii) specific for Glypican-1, CEA, CA19-9, mesothelin, PD-L1, telomerase, and any combination thereof; or
iii) a Glypican-1 antibody, a Glypican-1 tagging molecule, or a combination thereof.

5. The plurality of quantum dot nanoparticle conjugates of claim 1, wherein each quantum dot nanoparticle comprises a II-VI, III-V or I-III-VI material, or any alloy or doped derivative thereof.

6. The plurality of quantum dot nanoparticle conjugates of claim 1, wherein each quantum dot nanoparticle is associated with an emission spectrum ranging from about 350 nm to about 1000 nm.

7. The plurality of quantum dot nanoparticle conjugates of claim 1, wherein each quantum dot nanoparticle further comprises a cellular uptake enhancer, a tissue penetration enhancer, or a combination thereof.

8. A method of detecting exosomes comprising
i) contacting a plurality of quantum dot nanoparticle conjugates according to claim 1 with an exosome;
ii) exciting the plurality of quantum dot nanoparticle conjugates with a light source; and
iii) detecting light emission or light absorbance by the plurality of quantum dot nanoparticle conjugates.

9. The method of claim 8, wherein quantum dot nanoparticle is conjugated to a specific exosome-specific binding ligand based on the fluorescence wavelength of the quantum dot; optionally wherein the detection is performed in real-time.

10. The method of claim 8, wherein a unique emission code (a unique fingerprint) is obtained that may be associated with that specific type of exosome.

11. A method of detecting cancer comprising
i) contacting a plurality of quantum dot nanoparticle conjugates according to claim 1 with an exosome;
ii) exciting the plurality of quantum dot nanoparticle conjugates with a light source; and
iii) detecting light emission or light absorbance by the plurality of quantum dot nanoparticle conjugates.

12. The method of claim 11, wherein quantum dot nanoparticle is conjugated to a specific exosome-specific binding ligand based on the fluorescence wavelength of the quantum dot.

13. The method of claim 11, wherein a unique emission code (a unique fingerprint) is obtained that may be associated with the type of cancer that the exosome is released from.

14. The method of claim 11, wherein the cancer is pancreatic cancer, bladder cancer, lung cancer, or any combination thereof.

15. The method of claim 11, wherein the detection is performed:
i) *in vivo* or *in vitro*; or
ii) on a sample that is a fixed tissue or fluid sample or a sample that is a tissue or fluid sample in a live cell cultivated in a cell culture; or
iii) using a lateral flow assay device.
